# EUROPEAN PATENT SPECIFICATION

(11) **EP 1 581 477 B1**
(45) Date of publication and mention of the grant of the patent: **02.12.2009**
(21) Application number: 03799487.8
(22) Date of filing: 17.12.2003
(51) Int. Cl.: C07C 233/57, A61P 35/00, A61P 37/02, A61P 29/00, A61P 31/12, A61K 31/194

(54) **CYCLOALKENE DICARBOXYLIC ACID COMPOUNDS AS ANTI-INFLAMMATORY, IMMUNOMODULATORY AND ANTI-PROLIFERATORY AGENTS**
CYCLOALKEN-DICARBONSÄURE-VERBINDUNGEN ALS ENTZÜNDUNGSHEMMENDE, IMMUNMODULATORISCHE UND ANTIPROLIFERATIVE MITTEL
COMPOSES D'ACIDE DICARBOXYLIQUE DE CYCLOALCENE SERVANT D'AGENTS ANTI-INFLAMMATOIRES, D'IMMUNOMODULATION ET ANTI-PROLIFERATION

(30) Priority: 23.12.2002 DE 10260800
(43) Date of publication of application: 05.10.2005
(73) Proprietor: 4SC AG, 82152 Martinsried (DE)
(72) Inventor: LEBAN, Johann, 82110 Germering (DE); KRALIK, Martin, 6850 Dornbirn (AT)
(74) Representative: Kilger, Ute
(86) International application number: PCT/EP2003/014434
(87) International publication number: WO 2004/056746

(56) References cited:
- EP-A- 0 097 056
- WO-A-01/24785
- WO-A-99/65867
- WO-A-03/006424
- WO-A-03/006425
- WO-A-03/006443
- DE-A- 2 851 379
- DE-A- 2 921 002
- DE-A- 3 346 814
- US-A- 4 126 691
- CHEN S-F ET AL: "STRUCTURE-ACTIVITY RELATIONSHIP OF QUINOLINE CARBOXYLIC ACIDS. A NEW CLASS OF INHIBITORS OF DIHYDROOROTATE DEHYDROGENASE" BIOCHEMICAL PHARMACOLOGY, PERGAMON, OXFORD, GB, vol. 40, no. 4, 1990, pages 709-714, XP000900094 ISSN: 0006-2952
- THORSTENSSON, FREDRIK ET AL: "Synthesis of Novel Thrombin Inhibitors. Use of Ring-Closing Metathesis Reactions for Synthesis of P2 Cyclopentene - and Cyclohexenedicarboxylic Acid Derivatives" JOURNAL OF MEDICINAL CHEMISTRY, 46(7), 1165-1179, 2003, XP002274167
- DE JULIAN-ORTIZ JESUS V ET AL: "Virtual Combinatorial Syntheses and Computational Screening of New Potential Anti-Herpes Compounds" JOURNAL OF MEDICINAL CHEMISTRY, AMERICAN CHEMICAL SOCIETY. WASHINGTON, US, vol. 42, 1999, pages 3308-3314, XP002199074 ISSN: 0022-2623
- TAKEDA CHEMICAL INDUSTRIES ET AL: "Tetrahydrophthalamide derivatives" CHEMICAL ABSTRACTS + INDEXES, AMERICAN CHEMICAL SOCIETY. COLUMBUS, US, vol. 94, no. 25, 22 June 1981 (1981-06-22), XP002199076 ISSN: 0009-2258
- MATSUI ET AL: "N-Substituted-.DELTA.'-cyclopentene-1,2-d icarboxylic acid monoamides as herbicides" CHEMICAL ABSTRACTS + INDEXES, AMERICAN CHEMICAL SOCIETY. COLUMBUS, US, vol. 84, no. 5, 2 February 1976 (1976-02-02), XP002199075 ISSN: 0009-2258
- E.CAMPAIGNE: "synthesis of some ureidodihydrofurans and related pyrimidones as potential antimalarials" J.MED.CHEM, vol. 12, no. 2, 1969, pages 339-342, XP002278920

## Description

The present invention relates to novel compounds that can be used as antiinflammatory, immunomodulatory and antiproliferatory agents. In particular the invention refers to new compounds which inhibit dihydroorotate dehydrogenase (DHODH), a process for their manufacture, pharmaceutical compositions containing them and to their use in the manufacture of a medicament for the treatment and prevention of diseases, in particular their use in diseases where there is an advantage in inhibiting dihydroorotate dehydrogenase (DHODH).

Rheumatoid arthritis (RA) is a disease which is quite common especially among elder people. Its treatment with usual medications as for example non-steroid anti-inflammatory agents is not satisfactory. In view of the increasing ageing of the population, especially in the developed Western countries or in Japan the development of new medications for the treatment of RA is urgently required.

WO 99/38846 and EP 0 646 578 disclose compounds which are reported to be useful for treatment of RA.

A medicament against rheumatoid arthritis with a new mechanism of action, leflunomide, was recently put on the market by the company Aventis under the tradename ARAVA [EP 780128, WO 97/34600]. Leflunomide has immunomodulatory as well as anti-inflammatory properties [EP 217206, DE 2524929]. The mechanism of action is based upon the inhibition of dihydroorotate dehydrogenase (DHODH), an enzyme of the pyrimidine biosynthesis.

In the body, DHODH catalyzes the synthesis of pyrimidines, which are necessary for cell growth. An inhibition of DHODH inhibits the growth of (pathologically) fast proliferating cells, whereas cells which grow at normal speed may obtain their required pyrimidine bases from the normal metabolic cycle. The most important types of cells for the immuno response, the lymphocytes, use exclusively the synthesis of pyrimidines for their growth and react particularly sensitively to DHODH inhibition. Substances that inhibit the growth of lymphocytes are important medicaments for the treatment of auto-immuno diseases.

The DHODH inhibiting leflunomide (ARAVA) is the first medicament of this class of compounds (leflunomides) for the treatment of rheumatoid arthritis. WO 99/45926 is a further reference that discloses compounds which act as inhibitors of DHODH.

WO 01/85685 discloses heterocylic derivatives such as thiadiazolidindiones for the treatment of a disease in which GSK-3 is involved, including Alzheimer's disease or the non-dependent insulin diabetes mellitus, or hyperprofilerative disease such as cancer, arterosclerosis or restenosis.

N-phenyltetrahydroisoindolediones and N-phenyltetrahydropyridazinediones (US 5719104), 6-azolylcumarins (DE 3810706), arylheterocycles (EP 796845) and N-(heterocyclylphenyl) pyrroloimidazolsulfonamides (WO 97/15576) are described as herbicides.

In J. Org. Chem. 1985, 50 (14), 2450-2456, pyrrolo[3,4-d]imidazole ring systems are described to have fluorescence properties.

In EP 463444, WO 98/57937, EP 150034, Nucleosides & Nucleotides 1997, 16 (10 & 11), 2025-2033; Egyptian Journal of Pharmaceutical Sciences 1991, 32 (1-2), 331-339, Journal für Praktische Chemie 1991, 333 (4), 619-624, Archives of Pharmacal Research 1990, 13 (4), 338-341, Sulfur Letters 1988, 7 (4), 127-136, Synthesis 1988, 6 449-452, Sulfur Letters 1987, 7 (19), 19-24, Archiv der Pharmazie 1987, 320 (12), 1281-1283, Natl. Def. Med. Cent. 1983, 35 (1), 57-64 and Sch. Pharm. Sci. 1977, 97 (4), 410-415 a number of five membered aromatic ring systems fused to substituted maleimide are described.

It is an object of the present invention to provide alternative effective agents which can be used for the treatment of diseases which require the inhibition of DHODH.

Accordingly, a novel class of compounds with an inhibitory effect on DHODH, in particular human DHODH, was found.

The present invention is therefore directed to compounds of the general formula (I) 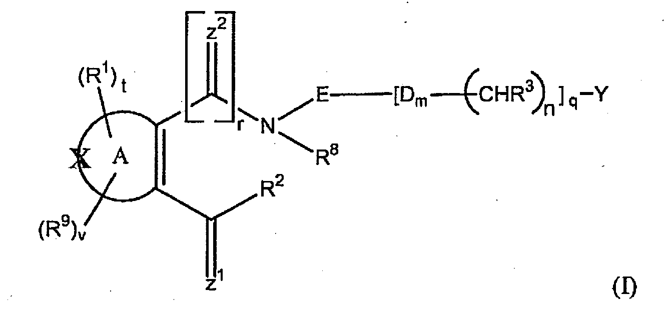 wherein
- A: is 1-cyclopentene-1,2-diyl
- D: is O, S, SO₂, NR⁴ or CH₂;
- Z¹ and Z²: are independent from each other O, S, or NR⁵ ;
- R¹: is independently -CO₂R", -SO₃H, -CONR*R", -CR"O, -SO₂-NR*R", - NO₂, -SO₂-R", -SO-R*, -CN, alkoxy, -OH, -SH, alkylthio, -NR"-CO₂-R', - NR"-CO-R*, -NR"-SO₂-R', -O-CO-R*, -O-CO₂-R*, -O-CO-NR*R"; cycloalkyl, alkylamino, hydroxyalkylamino, aryl, or heteroaryl;
- R⁹: is independently H, halogen, haloalkyl, haloalkyloxy or alkyl;
- R*: is independently H, alkyl, cycloalkyl, aminoalkyl, alkoxy, -OH, -SH, alkylthio, hydroxyalkyl, haloalkyl, haloalkyloxy, aryl or heteroaryl;
- R": is independently hydrogen, haloalkyl, hydroxyalkyl, alkyl, cycloalkyl, aryl, heteroaryl or aminoalkyl;
- R²: is H, OR⁶, or NHR⁷;
- R³: is H; alkyl, cycloalkyl, aryl, arylalkyl, alkoxy, O-aryl; O-cycloalkyl, halogen, aminoalkyl, alkylamino, hydroxylamino, hydroxylalkyl, haloalkyl, haloalkyloxy, heteroaryl, alkylthio, S-aryl, or S-cycloalkyl;
- R⁴: is H, alkyl, cycloalkyl, aryl, or heteroaryl;
- R⁵: is H, OH, alkoxy, O-aryl, alkyl, or aryl;
- R⁶: is H, alkyl, cycloalkyl, aryl, heteroaryl, arylalkyl, alkylaryl, alkoxyalkyl, acylmethyl, (acyloxy)alkyl, non-symmetrical (acyloxy)alkyldiester, or dialkylphosphate;
- R⁷: is H, alkyl, aryl, alkoxy, O-aryl, cycloalkyl, or O-cycloalkyl;
- R⁸: is hydrogen or alkyl;
- E: is a substituted or unsubstituted phenyl ring system;
- Y: is a substituted or unsubstituted phenyl ring system
- m: is 0 or 1;
- n: is 0 or 1;
- p: is 0 or 1;
- r: is 0 or 1;
- q: is 0 or 1;
- t: is 1 to 3; and
- v: is 0 to 3;

The present invention is also directed to compounds of the general formule (Ia) 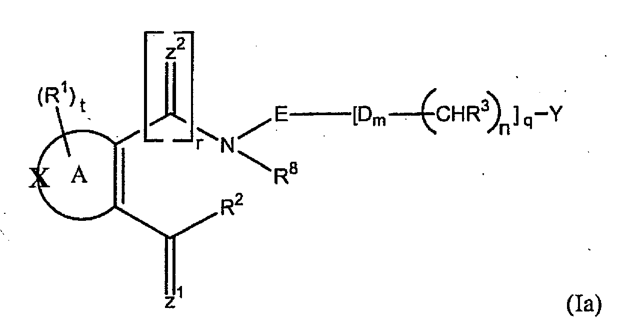 wherein
- A: is 1-cyclopentene-1,2-diyl;
- D: is O, S, SO₂, NR⁴, or CH₂;
- Z¹ and Z²: are independent from each other O, S, or NR⁵;
- R¹: is independently H, halogen, haloalkyl, haloalkyloxy -CO₂R", -SO₃H, -OH, -CONR*R", -CR"O, -SO₂-NR*R", -NO₂, -SO₂-R", -SO-R*, -CN, alkoxy, alkylthio, aryl, -NR"-CO₂-R', -NR"-CO-R*, -NR"-SO₂-R', -O-CO-R*, - O-CO₂-R*, -O-CO-NR*R"; cycloalkyl, alkylamino, hydroxyalkylamino, - SH, heteroaryl, or alkyl;
- R*: is independently H, alkyl, cycloalkyl, aminoalkyl, alkoxy, -OH, -SH, alkylthio, hydroxyalkyl, haloalkyl, haloalkyloxy, aryl or heteroaryl;
- R": is independently hydrogen, haloalkyl, hydroxyalkyl, alkyl, cycloalkyl, aryl, heteroaryl or aminoalkyl;
- R²: is NHOH or R² together with the nitrogen atom which is attached to R⁸ form a 5 or 6 membered heterocyclic ring with the proviso that R² is -[CR₂]ₛ and R⁸ is absent;
- R³: is H, alkyl, cycloalkyl, aryl, alkoxy, O-aryl; O-cycloalkyl, halogen, aminoalkyl, alkylamino, hydroxylamino, hydroxylalkyl, haloalkyloxy, heteroaryl, alkylthio, S-aryl; S-cycloalkyl, arylalkyl, or haloalkyl;
- R⁴: is H, alkyl, cycloalkyl, aryl or heteroaryl;
- R⁵: is H, OH, alkoxy, O-aryl, alkyl or aryl;
- R⁸: is hydrogen, or alkyl;
- E: is a substituted or unsubstituted phenyl ring system;
- Y: is a substituted or unsubstituted phenyl ring system
- m: is 0 or 1;
- n: is 0 or 1;
- p: is 0 or 1;
- r: is 0 or 1;
- q: is 0 or 1;
- s: is 0 to 2; and
- t: is 0 to 3;
an alkyl group, if not stated otherwise, denotes a linear or branched C₁-C₆-alkyl, preferably a linear or branched chain of one to five carbon atoms, a linear or branched C₁-C₆-alkenyl or a linear or branched C₁-C₆-alkinyl group, which can optionally be substituted by one or more substituents R', preferably by halogen;
the C₁-C₆-alkyl, C₁-C₆-alkenyl and C₁-C₆ alkinyl residue may be selected from the group comprising -CH₃, -C₂H₅, -CH=CH₂, -C=CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -C₂(R')₅, -CH₂-C(R')₃, -C₃(R')₇, -C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C=CH, -C≡H=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C=CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, - C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;
R' is independently H, -CO₂R", -CONHR", -CR"O, -SO₂NR", NR"-CO-haloalkyl, -NO₂, -NR"-SO₂-haloalkyl, -NR"-SO₂-alkyl, -SO₂-alkyl, -NR"-CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;
R" is independently hydrogen, haloalkyl, hydroxyalkyl, alkyl, cycloalkyl, aryl, heteroaryl or aminoalkyl;
a cycloalkyl group denotes a non-aromatic ring system containing three to eight carbon atoms, preferably four to eight carbon atoms, wherein one or more of the carbon atoms in the ring can be substituted by a group X, X being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅;
an alkoxy group denotes an O-alkyl group, the alkyl group being as defined above; the alkoxy group is preferably a methoxy, ethoxy, isopropoxy, t-butoxy or pentoxy group;
an alkylthio group denotes an S-alkyl group, the alkyl group being as defined above.
an haloalkyl group denotes an alkyl group which is substituted by one to five halogen atoms, the alkyl group being as defined above; the haloalkyl group is preferably a -C(R¹⁰)₃, -CR¹⁰(R¹⁰')₂, -CR¹⁰(R¹⁰')R^{10"}, -C₂(R¹⁰)₅, -CH₂-C(R¹⁰)₃, -CH₂-CR¹⁰(R^{10'})_{2'} -CH₂-CR¹⁰(R¹⁰)R¹⁰", -C₃(R¹⁰)₇ or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"} -OC₂(R¹⁰)₅, -OCH₂-C(R¹⁰)₃, -OCH₂-CR¹⁰(R^{10'})₂, -OCH₂-CR¹⁰(R¹⁰)R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is chlorine, bromine, fluorine or iodine, fluorine being preferred;
an aryl group preferably denotes an aromatic group having five to fifteen carbon atoms, which can optionally be substituted by one or more substituents R', where R' is as defined above; the aryl group is preferably a phenyl group, -CH₂Ph, -C₂H₄Ph, -CH=CH-Ph, - C≡C-Ph, -o-C₆H₄- R', m-C₆H₄- R', p-C₆H₄- R', -o-CH₂-C₆H₄ R', -m-CH₂-C₆H₄-R', -p-CH₂-C₆H₄- R';
a heteroaryl group denotes a 5- or 6-membered heterocyclic group which contains at least one heteroatom like O, N, S. This heterocyclic group can be fused to another ring. For example, this group can be selected from a thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yi, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, 1H-tetrazol-2-yl, 1H-tetrazol-3-yl, tetrazolyl, indolyl, indolinyl, benzo-[b]-furanyl, benzo[b]thiophenyl, benzimidazolyl, benzothiazolyl, quinazolinyl, quinoxazolinyl; or preferably quinolinyl, tetrahydroquinolinyl, isoquinolinyl, tetrahydroisoquinolinyl group. This heterocyclic group can optionally be substituted by one or more substituents R', where R' is as defined above.

The meaning of E includes optional by one or more substituents R' substituted

The invention also provides a pharmaceutical composition comprising a compound of formula (I) or of formula (Ia) in free form or in the form of pharmaceutically acceptable salts together with a pharmaceutically acceptable diluent or carrier therefore.

Also herein described is a method for the treatment or prophylaxis of a condition where there is an advantage in inhibiting dihydroorotate dehydrogenase (DHODH) which comprises the administration of an effective amount of a compound of formula (I) or, (Ia), and physiologically acceptable salts.

The invention is also directed to the use of compounds of the formula (I), or (Ia) and of their pharmacologically tolerable salts for the production of a medicament for the prevention and treatment of diseases, where inhibition of the pyrimidine biosynthesis is of benefit.

In addition, the present invention provides methods for preparing the compounds of the invention such as compounds of formula (I), or of formula (Ia).

The compounds of formula (I), or of formula (Ia) may be obtained via various methods. In preferred embodiments of the methods of the invention the following methods of synthesis are used.

For the synthesis of dicarboxylic acids substituted in or on the ring system two methods were used.

### Method 1:

The synthesis of dicarboxylic acid dimethylester is described in WO 02/07655.
This dicarboxylic acid dimethyl ester can be substituted on the ring system as descibed by T. Harrison et.al., Tetrahedron Vol. 45, No.16, 1989, 5247-5262. This dicarboxylic acid dimethyl ester can then be converted into the corresponding acid anhydride.

Method 2: Dicarboxylic acids substituted in or on the ring system can also be synthesized in general via the cyanhydrine synthesis [Shwu-Jiüan Lee et.al., Bull. Inst. Chem. Academia Sinica Number 40, (1993), 1-10 or B. R. Baker at al., J. Org. Chem. 13, 1948, 123-133; and B. R. Baker at al., J. Org. Chem. 12, 1947, 328-332; L. A. Paquette et. al., J. Am: Chem. Soc. 97, (1975), 6124-6134]. This dicarboxylic acid can then be converted into the corresponding acid anhydride.
These anhydrides may then be reacted with the corresponding amines to the desired amides of formula (I), or of formula (Ia). This reaction steps are analog to the reaction steps described in WO 02/07655.

The compounds of formula (I), or of formula (Ia), in each case [r = 0] can be synthesized analog to the four methods described in WO 02/07655.

The invention also provides methods for preparing compounds of formula (Ia) wherein R² together with the nitrogen atom which is attached to R⁸ form a 6 membered heterocylic ring system.
The respective substituted aniline of formula (Ia) prepared in analogy to WO 02/07655 was deprotonated with sodium hydride in tetrahydrofuran and alkylated with 2-bromomethyl-[1,3]dioxolane. Starting from 2-(carbethoxy)cyclopentanone, 2-(aminocarbonyl)-2-cyclopentene-1-ones were synthesized by amidation using the corresponding N-alkyl aniline and 4-dimethylaminopyridine, followed by oxidation with lead tetraacetate in the presence of catalytic amounts of copper(II) acetate (A. G. Schulz et al., Tetrahedron Lett. 34, 1993, 3021-3024). Deacetalization at the aniline side chain set free an aldehydic group, which was used for a Stetter reaction by reversion of polarity with thiazolium salts and subsequent 1,4-addition (H. Stetter, Angew. Chem. 88, 1976, 695-704).

In addition, the present invention provides methods for preparing the desired hydroxamic acides of formula (Ia).
One method for the synthesis of compounds of formula (Ia) comprises the conversion of an acid to the corresponding acid chloride and reacting the acid chloride with hydroxylamine (Watanabe et al., 1989, J. Org. Chem., 54, 17, 4088-4097; Shishido et al., 1992, J. Org. Chem., 57,10,2876-2883).
Other methods for the preparation of compounds of formula (Ia) are described by Woo et al., 2002, J. Med. Chem. 45, 2877-2885; Knorr et al., 1989, Tetrahedron Lett., 30, 1927-1930, Carpino, 1993, J. Am. Chem. Soc., 115, 4397-4398 and Albericio et.al., 1998, J. Org. Chem., 63, 9678-9683.

Another method for the preparation of compounds of formula (Ia) is the reaction of the corresponding ester with hydroxylamine as described by Stowell et al., 1995, J. Med. Chem., 38, 8; 1411-1413.

The synthesis of amides of formula (Ia) is described by J. Zabicky in "The Chemistry of Amides", in the serial of S. Patai (ed.), "The Chemistry of Functional Groups", John Wiley & Sons, 1975, p. 74-131. Methods for preparing thioamides are described in Houben-Weyl, J. Falbe (ed.), G. Thieme Verlag, vol. E5, p. 1219-59. Methods for preparing sulfamides are described by Caldwell et al., J. Am. Chem. Soc. 1944, 66, 1479-82, or by Flynn et al., Med. Chem. Res., 1998, 8, 219-43 and Dziadulewicz et al., Bioorg. Med. Chem. Lett. 2001,11,5, 705-10.

In another preferred embodiment, in the compounds of formula (I) 3 position on the 1-cyclobuten-1,2-diyl ring system.

In another preferred embodiment, in the compounds of formula (I) 5 position on the 1-cyclobuten-1,2-diyl ring system.

In the compounds of formula (I), or of formula (Ia) R¹ is preferably OH, OCH₃, SH, CO₂H or SO₃H or tetrazole. In the compounds of formula (I) R⁹ is preferably H.

In the compounds of formula (I) R² is H, OR⁶, preferably OH or OR⁶.
In the compounds of formula (Ia) R² is preferably NHOH.

In preferred embodiment, in the compounds of formula (I) R⁶ is benzoyloxymethyl, isobutyryloxymethyl, 4-amino-butyryloxymethyl, butyryloxymethyl, 1-(butyryloxy)ethyl, 1-(butyryloxy)-2,2-dimethylpropyl, 1-diethyl-phosphonooxyethyl, 2-(2-methoxyethoxy)-acetyloxymethyl, p-aminobenzoyl-methyl, nicotinyloxymethyl, pivalyloxymethyl, glutaryloxymethyl, [2-(2-methoxy-ethoxy)ethoxy]-acetyloxymethyl, 2-(morpholine-4-yl)-ethyl, 1-diethyl-phosphono-oxymethyl.

In the compounds of formula (I), or of formula (Ia) R³ is is H, alkyl, cycloalkyl, aryl, alkoxy, O-aryl; O-cycloalkyl, halogen, aminoalkyl, alkylamino, hydroxylamino, haloalkyl, hydroxylalkyl, haloalkyloxy, heteroaryl, alkylthio, S-aryl; S-cycloalkyl, arylalkyl, preferably H.

In the compounds of formula (I), or of formula (Ia) R⁴ is H, alkyl, cycloalkyl, aryl or heteroaryl, preferably H.

In formula (I), or in formula (Ia) R⁸ is H or alkyl, preferably H or methyl.

In formula (I), or formula (Ia) Z¹ and Z² are independent from each other O, S, or NR⁵, preferably both are O.

In formula (I), or formula (Ia) Y is an optionally substituted phenyl.

In formula (I), or formula (Ia) E is preferably an optionally by one or more substituents R' substituted phenyl.

In a preferred embodiment of the present invention in compounds of formula (I), or of formula (Ia) E is an optionally by one or more substituents R' substituted phenyl, or an optionally by one or more substituents R' substituted cycloalkyl.

In formula (I), or formula (Ia), preferred substituents R' are nitro, halogen, alkoxy, haloalkyl, haloalkyloxy, heteroaryl, alkyl or aryl, more preferably R' is Br, F, Cl, CF₃, OCF₃, ethoxy or methoxy.

In formula (I), or formula (Ia) preferred heteroaryl groups are imidazoyl, thienyl, furanyl, pyridyl, pyrimidyl, pyranyl, pyrazolyl, pyrazinyl, thiazolyl; 1H-tetrazol-2-yl, 1H-tetrazol-3-yl, or oxazolyl.

In formula (I) t is preferably 1 or 2.
In formula (Ia) t is preferably 0, 1 or 2.
In formula (I) v is preferably 0 or 1.
In formula (Ia) s is preferably 0 or 1

In the compounds of formula (I), or of formula (Ia) D is O, S, SO₂, NR⁴, or CH₂. D is preferably S or more preferably O, when m = 1.

In other preferred embodiment, in the compounds of formula (I), or of formula (Ia) m and q are zero and Y is an optionally by one or more substituents R' substituted phenyl or more preferably an optionally by one or more F, Cl, methoxy, ethoxy, CF₃, or OCF₃ substituted phenyl.

In particular preferred embodiment of the invention, in compounds of formula (I), or of formula (Ia) q=0, t=1, and E is phenyl which is either unsubstituted or substituted with Cl, F and/or CF₃ or OCF₃.

In further particularly preferred embodiment, in compounds of formula (I) or of formula (Ia), D=O (thus m=1), R³ is H (thus n=1), q=1, t=1, and E is phenylene which is either unsubstituted or substituted with Cl, F and/or CF₃ or OCF₃, and Y is phenyl which is also either unsubstituted or substituted with Cl, F and/or CF₃, methoxy, ethoxy, or OCF₃.

In further particularly preferred embodiment, in compounds of formula (I) or of formula (Ia), D=O (thus m=1), n=0, q=1, t = 1, and E is phenylene which is either unsubstituted or substituted with Cl, F and/or CF₃ or OCF₃, and Y is phenyl which is also either unsubstituted or substituted with Cl, F and/or CF₃, methoxy, ethoxy, or OCF₃.

In further particularly preferred embodiment, in compounds of formula (I) of formula (Ia), of formula (III), or of formula (IV), D=S (thus m=1), n=0, q=1, t=1, and E is phenylene which is either unsubstituted or substituted with Cl, F and/or CF₃ or OCF₃, and Y is phenyl which is also either unsubstituted or substituted with methoxy, ethoxy, Cl, F and/or C3 or OCF₃.

In formula (I), or formula (Ia), q is 0 or 1. If q is 1 and n is 0 or 1, D is preferably O (thus m=1).

In particular preferred embodiments of the invention, in compounds of formula (I), t=1, Z¹=O, Z²=O (thus r=1), E is phenylene which is either unsubstituted or substituted with Cl, F and/or CF₃ or OCF₃, and Y is phenyl which is also either unsubstituted or substituted with methoxy, ethoxy, Cl, F and/or CF₃ or OCF₃.

The compounds of the formula (I), or of formula (Ia), to be used according to the invention can form salts with inorganic or organic acids or bases. Examples of such salts are, for example, alkali metal salts, in particular sodium and potassium salts, or ammonium salts.

The compounds of the present invention can be used for a variety of human and animal diseases, preferably human diseases, where inhibition of the pyrimidine metabolism is beneficial. Such diseases are:
- fibrosis, uveitis, rhinitis, asthma or arthropathy, in particular, arthrosis
- all forms of rheumatism
- acute immunological events and disorders such as sepsis, septic shock, endotoxic shock, Gram-negative sepsis, toxic shock sydrome, acute respiratory distress syndrome, stroke, reperfusion injury, CNS injury, serious forms of allergy, graft versus host and host versus graft reactions, alzheimer's or pyresis, restenosis, chronic pulmonary inflammatory disease, silicosis, pulmonary sarcosis, bone resorption disease. These immunological events also include a desired modulation and suppression of the immune system;
- all types of autoimmune diseases, in particular rheumatoid arthritis, rheumatoid spondylitis, osteoarthritis, gouty arthritis, multiple sclerosis, insulin dependent diabetes mellitus and non-insulin dependent diabetes, and lupus erythematoidis, ulcerative colitis, Morbus Crohn, inflammatory bowel disease, as well as other chronic inflammations, chronic diarrhea;
- dermatological disorders such as psoriasis
- progressive retinal atrophy
- all kinds of infections including opportunistic infections.

The compounds according to the invention and medicaments prepared therewith are generally useful for the treatment of cell proliferation disorders, for the treatment or prophylaxis, immunological diseases and conditions (as for instance inflammatory disease, neuroimmunological diseases, autoimmune diseases or other).

The compounds of the present invention are also useful for the development of immunomodulatory and anti-inflammatory medicaments or, more generally, for the treatment of diseases where the inhibition of the pyrimidine biosynthesis is beneficial.

The compounds of the present invention are also useful for the treatment of diseases which are caused by malignant cell proliferation, such as all forms of hematological and solid cancer. Therefore the compounds according to the invention and medicaments prepared therewith are generally useful for regulating cell activation, cell proliferation, cell survival, cell differentiation, cell cycle, cell maturation and cell death or to induce systemic changes in metabolism such as changes in sugar, lipid or protein metabolism. They can also be used to support cell generation poiesis, including blood cell growth and generation (prohematopoietic effect) after depletion or destruction of cells, as caused by, for example, toxic agents, radiation, immunotherapy, growth defects, malnutrition, malabsorption, immune dysregulation, anemia and the like or to provide a therapeutic control of tissue generation and degradation, and therapeutic modification of cell and tissue maintenance and blood cell homeostasis.

These diseases and conditions include but are not limited to cancer as hematological (e.g. leukemia, lymphoma, myeloma) or solid tumors (for example breast, prostate, liver, bladder, lung, esophageal, stomach, colorectal, genitourinary, gastrointestinal, skin, pancreatic, brain, uterine, colon, head and neck, and ovarian, melanoma, astrocytoma, small cell lung cancer, glioma, basal and squameous cell carcinoma, sarcomas as Kaposi's sarcoma and osteosarcoma), treatment of disorders involving T-cells such as aplastic anemia and DiGeorge syndrome, Graves' disease.

Leflunomide, was previously found to inhibit HCMV replication in cell culture. Ocular herpes is the most common couse of infectious blindness in the developed world. There are about 50.000 cases per year in the US alone, of which 90% are recurences of initial infections. Recurrences are treated with antivirals and corticosteroids. Cytomegalovirus another herpes virus is a common couse of retinal damage and blindness in patients with aids. The compounds of the present invention can be used alone or in combination with other antiviral compounds such as Ganciclovir and Foscarnet to treat such diseases.

The compounds of the present invention can further be used for diseases that are caused by protozoal infestations in humans and animals. Such veterinary and human pathogenic protozoas are preferably intracellular active parasites of the phylum Apicomplexa or Sarcomastigophora, especially Trypanosoma, Plasmodia, Leishmania, Babesia and Theileria, Cryptosporidia, Sacrocystida, Amoebia, Coccidia and Trichomonadia. These active substances or corresponding drugs are especially suitable for the treatment of Malaria tropica, caused by *Plasmodium falciparum,* Malaria tertiana, caused by *Plasmodium vivax* or *Plasmodium ovale* and for the treatment of Malaria quartana, caused by *Plasmodium malariae.* They are also suitable for the treatment of Toxoplasmosis, caused by *Toxoplasma gondii,* Coccidiosis, caused for instance by *Isospora belli,* intestinal Sarcosporidiosis, caused by *Sarcocystis suihominis;* dysentery caused by *Entamoeba histolytica,* Cryptosporidiosis, caused by *Cryptosporidium parvum,* Chargas' disease, caused by *Trypanosoma cruzi,* sleeping sickness, caused by *Trypanosoma brucei rhodesiense* or *gambiense,* the cutaneous and visceral as well as other forms of Leishmaniosis. They are also suitable for the treatment of animals infected by veterinary pathogenic protozoa, like *Theileria parva,* the pathogen causing bovine East coast fever, *Trypanosoma congolense congolense* or *Trypanosoma vivax vivax, Typanosoma brucei brucei,* pathogens causing Nagana cattle disease in Africa, *Trypanosoma brucei evansi* causing Surra, *Babesia bigemina,* the pathogen causing Texas fever in cattle and buffalos, *Babesia bovis,* the pathogen causing european bovine Babesiosis as well as Babesiosis in dogs, cats and sheep, *Sarcocystis ovicanis* and *ovifelis* pathogens causing Sarcocystiosis in sheep, cattle and pigs, Cryptosporidia, pathogens causing Cryptosporidioses in cattle and birds, Eimeria and Isospora species, pathogens causing Coccidiosis in rabbits, cattle, sheep, goats, pigs and birds, especially in chickens and turkeys. The use of the compounds of the present invention is preferred in particular for the treatment of Coccidiosis or Malaria infections, or for the preparation of a drug or feed stuff for the treatment of these diseases. This treatment can be prophylactic or curative. In the treatment of malaria, the compounds of the present invention may be combined with other anti-malaria agents.

The compounds of the present invention can further be used for viral infections or other infections caused for instance by *Pneumocystis carinii.*

The compounds of formula (I), or formula (Ia), and their pharmacologically acceptable salts can be administered to animals, preferably to mammals, and in particular to humans, dogs and chickens as therapeutics per se, as mixtures with one another or in the form of pharmaceutical preparations which allow enteral or parenteral use and which as active constituent contain an effective dose of at least one compound of the formula (I), or formula (Ia), or a salt thereof, in addition to customary pharmaceutically innocuous excipients and additives. The compounds of formula (I), or of formula (Ia), can also be administered in form of their salts, which are obtainable by reacting the respective compounds with physiologically acceptable acids and bases.

The therapeutics can be administered orally, e.g. in the form of pills, tablets, coated tablets, sugar coated tablets, hard and soft gelatin capsules, solutions, syrups, emulsions or suspensions or as aerosol mixtures. Administration, however, can also be carried out rectally, e.g. in the form of suppositories, or parenterally, e.g. in the form of injections or infusions, or percutaneously, e.g. in the form of ointments, creams or tinctures.

In addition to the active compounds of formula (I), or of formula (Ia), the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain two or more compounds of the formula (I), or of formula (Ia), or their pharmacologically acceptable salts and also other therapeutically active substances.

Thus, the compounds of the present invention can be used in the form of one substance alone or in combination with other active compounds - for example with medicaments already known for the treatment of the aforementioned diseases, whereby in the latter case a favorable additive, amplifying effect is hoticed. Suitable amounts to be administered to humans range from 5 to 500 mg.

To prepare the pharmaceutical preparations, pharmaceutically inert inorganic or organic excipients can be used. To prepare pills, tablets, coated tablets and hard gelatin capsules, for example, lactose, corn starch or derivatives thereof, talc, stearic acid or its salts, etc. can be used. Excipients for soft gelatin capsules and suppositories are, for example, fats, waxes, semi-solid and liquid polyols, natural or hardened oils etc. Suitable excipients for the production of solutions and syrups are, for example, water, sucrose, invert sugar, glucose, polyols etc. Suitable excipients for the production of injection solutions are, for example, water, alcohols, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 to 100 mg/kg animal body weight preferably 1 to 50 mg/kg. Suitable dosage rates for larger mammals, for example humans, are of the order of from about 10 mg to 3 g/day, conveniently administered once, in divided doses 2 to 4 times a day, or in sustained release form.

In general, a daily dose of approximately 10 mg to 5000 mg, preferably 50 to 500 mg, per human individual is appropriate in the case of the oral administration which is the preferred form of administration according to the invention. In the case of other administration forms too, the daily dose is in similar ranges.

The compounds of formula (I), or of formula (Ia) can also be used in the form of a precursor (prodrug) or a suitably modified form, that releases the active compound *in vivo.* Such precursors such as the preferred embodiments of R⁶ or R¹ can be obtained for example by masking the free acid group with an ester group, which is then in turn transformed into the free acid group in vivo [F. W. Sum et. al. Bioorg. & Med. Chem. Lett. 9 (1999), 1921-1926; Ada Rephaeli et. al. Drug Development Research 50 (2000) 379-391; H. Ishikawa, Current Med. Chem. 6 (1999), 575-597]. Further precursors for the preferred embodiments of R⁶ or R¹ is tetrazole, another metabolism-resistant isosteric replacements for the carboxylic acid group as described by J. Herr, Bioorg. & Med. Chem. Lett. 10 (2002), 3379-3393. Other precursors for the preferred embodiments of R⁵ can be obtained for example by masking the amidine with an hydroxy group, which is then in turn transformed into the free amidine *in vivo* [R.M. Scarborough, J. Med. Chem. 43, 19, (2000), 3454-3473].

### Examples

### 1. Synthesis of cyclopent-1-ene-1,2,3-tricarboxylic acid monoamide and cyclopent-1-ene-1,2,5-tricarboxylic acid monoamide and derivatives

A solution of cyclopent-1-ene-1,2-dicarboxylic acid methylester (1 eq, 1mmol) dissolved in chloroform (3ml) was heated at 70°C under reflux irradiated with a 300 W sunlamp. N-bromsuccinimide (1.05 eq) was added in small portions under heating and irradiating over a periode of 3 h. Heating and irradiation was continued for 2 h. After the reaction was finished (monitored by TLC) the mixture was cooled to room temperatur, the solvent was removed in vacuum and the residue was purified by flash chromatographie using a hexane/ethylacetat gradient to give 3-Bromo-cyclopent-1-ene-1,2-dicarboxylic acid dimethyl ester in a yield between 60 and 80 %.
3-Bromo-cyclopent-1-ene-1,2-dicarboxylic acid dimethyl ester was dissolved in acetonitril under inert conditions. Tetraethylammoniumcyanide (1.1 eq) was added in one portion.
The mixture was stirred under inert gas until the reaction was finished (2 h monitored by TLC). The solvent was removed in vacuum and the residue was purified by flash chromato-graphie using a hexane/ ethylacetat gradient to give 3-Cyano-cyclopent-1-ene-1,2-dicarboxylic acid dimethyl ester in 25 % yield.
3-Cyano-cyclopent-1-ene-1,2-dicarboaylic acid dimethyl ester was dissolved in concentrated hydrochloric acid and refluxed for 4 h. The solvent was removed in vacuum.
The resulting residue was purified by HPLC to give the product in 50 % yield Cyclopent-1-ene-1,2,3-tricarboxylic acid was suspended in acetic anhydride and stirred for 8 h. The solvent was removed in vacuum and the resulting crude product was used without further purification.
The resulting anhydrid (1eq, 1mmol) was dissolved in dichlormethane (3ml) and the 4-aminobiphenyl derivate (1eq, 1mmol) was add to the solution. After stirring for 18 h at room temperatures the solvent was removed in vacuum the residue was purified by HPLC to give the cyclopent-1-ene-1,2,3-tricarboxylic acid monoamide and cyclopent-1-ene-1,2,5-tricarboxylic acid monoamide derivative.

### 2. Synthesis of cyclopent-1-ene-1,2-dicarboxylic acid 2-hydroxyamide 1-monoamide derivate

A solution, of cyclopent-1-ene-1;2-dicarboxylic acide monoamide- (1equ, 1mmol) and TBTU (1eq, 1mmol) in dimethylformamide (3ml) was stirred for 10 minutes at room temperature. Afterwards O-t-butylhydroxylamine hydrochloride (1eq, 1mmol) and N,N-diisopropylethylamine (1eq, 1mmol) was added and the solution was stirred for 18h at room temperatures. The solvent was removed in vacuum and the residue was purified by HPLC to give the pure product.

### 3. Synthesis of 3-hydroxy-cyclopentene-dicarboxylic acide-monoamide and 5-hydroxy-cyclopentene-dicarboxylic acide-monoamide

A solution of 5,6-dihydro-4H-cyclopenta[c]furan-1,3-dione (1 eq, 1 mmol) in chloroform (3 ml) was heated at 70°C under reflux. During the solution was irradiated with a 300W sunlamp, N-bromosuccinimide (1,2 eq, 1,2 mmol) was added in small portions. After 1h the solution was cooled to room temperature, and the solvent was removed in vacuum. The residue was dissolved in dichloromethane (3 ml) and a 4-amino-biphenyl-derivate (1 eq, 1 mmol) was added. The solution was stirred at room temperature for 18h. The solvent was removed in vacuum and the residue was dissolved in acetonitrile/water and purified by HPLC to give the 3-hydroxy-cyclopentene-dicarboxylicacide-monoamide and 5-hydroxy-cyclopentene-dicarboxylic acide-monoamide

### 4. Synthesis cyclopentenedicarboxylic acid 2-monoamide 1-alkyl ester

5,6-Dihydro-4H-cyclopenta[c]furan-1,3-dione (1 eq, 1mmol) was dissolved in the respective alcohol (3ml) and stirred for 18h at room temperature to optain the desired mono alkyl ester. Afterwards the solvent was removed in vakuum and the residue was used without further purification in the next step.
Cyclopent-1-ene-1,2-dicarboxylic acid monoalkyl ester (1eq, 1mmol) was dissolved in dichlormethylen and thionylchlorid (1,2 eq, 1,2 mmol) was add dropwise under ice cooling. The mixture was stirred for 1h at room temperature and the solvent was removed at vacuum. This product was also used without a purification in the next step. 2-Chlorocarbonyl-cyclopent-1-enecarboxylic acid alkyl ester was solved in dichlormethylen (3 ml) and the respective 4-amino-biphenyl was added in one portion. The mixture was stirred for 18h at room temperature and afterwards the solvent was removed at vakuum. The product was purified by recristallisation.

### Example 1: 3-(3-Fluoro-3'-methoxy-biphenyl-4-ylcarbamoyl)-cyclopent-2-ene-1,2-dicarboxylic acid LC/(+)-ESI-MS: m/z = 400 [M+H]⁺

### Example 2: 2-(3-Fluoro-3'-methoxy-biphenyl-4-ylcarbamoyl)-cyclopent-1-ene-1,3-dicarboxylic acid LC/(+)-ESI-MS: m/z-= 400 [M+H]⁺

### Example 3: 2-(3-Fluoro-3'-methoxy-biphenyl-4-ylcarbamoyl)-cyclopent-1-enecarboxylic acid methyl esterLC/(+)-ESI-MS: m/z = 370 [M+H]⁺

### Example 4: Cyclopent-1-ene-1,2-dicarboxylic acid 1-[(3-fluoro-3'-methoxy-biphenyl-4-yl)-amide] 2-hydroxyamide LC/(+)-ESI-MS: m/z = 371 [M+H]⁺

### Example 5: 3-Hydroxy-2-(2,3,5,6-tetrafluoro-3'-trifluoromethoxy-biphenyl-4-ylcarbamoyl)-cyclopent-1-enecarboxylic acid LC/(+)-ESI-MS: m/z = 480 [M+H]⁺

### Example 6: 5-Hydroxy-2-(2,3,5,6-tetrafluoro-3'-trifluoromethoxy-biphenyl-4-ylcarbamoyl)-cyclopent-1-enecarboxylic acid LC/(+)-ESI-MS: m/z = 480 [M+H]⁺

### Example 7:2-(3'-Ethoxy-3,5-difluoro-biphenyl-4-ylcarbamoyl)-3-hydroxy-cyclopent-1-enecarboxylic acid LC/(+)-ESI-MS: m/z = 404 [M+H]⁺

### Example 8:2-(3'-Ethoxy-3,5-difluoro-biphenyl-4-ylcarbamoyl)-5-hydroxy-cyclopent-1-enecarboxylic acid LC/(+)-ESI-MS: m/z = 404 [M+H]⁺

### Example 9: 2-(1',3'di-methoxy-3,5-difluoro-biphenyl-4-ylcarbamoyl)-3-hydroxy-cyclopent-1-enecarboxylic acid LC/(+)-ESI-MS: m/z = 420 [M+H]⁺

### Example 10: 2-(1',3'di-methoxy-3,5-difluoro-biphenyl-4-ylcarbamoyl)-5-hydroxy-cyclopent-1-enecarboxylic acid LC/(+)-ESI-MS: m/z = 420 [M+H]⁺

### Example 11: 3-Hydroxy-2-(3,5,2'-trifluoro-biphenyl-4-ylcarbamoyl)-cyclopent-1-enecarboxylic acid LC/(+)-ESI-MS: m/z = 378 [M+H]⁺

### Example 12: 5-Hydroxy-2-(3,5,2'-trifluoro-biphenyl-4-ylcarbamoyl)-cyclopent-l-enecarboxylic acid LC/(+)-ESI-MS: m/z = 378 [M+H]⁺

### Example 13: 2-(2-Chloro-2'-methoxy-biphenyl-4-ylcarbamoyl)-3-hydroxy-cyclopent-1-enecarboxylic acid LC/(+)-ESI-MS: m/z = 388 [M+H]⁺

### Example 14: 2-(2-Chloro-2'-methoxy-biphenyl-4-ylcarbamoyl)-5-hydroxy-cyclopent-1-enecarboxylic acid LC/(±)-ESI-MS: m/z = 388 [M+H]⁺

### Example 15: 2-(2'-Chloro-3,5-difluoro-biphenyl-4-ylcarbamoyl)-3-hydroxy-cyclopent-1-enecarboxylic acid LC/(+)-ESI-MS: m/z = 394 [M+H]⁺

### Example 16: 2-(2'-Chloro-3,5-difluoro-biphenyl-4-ylcarbamoyl)-5-hydroxy-cyclopent-1-enecarboxylic acid LC/(+)-ESI-MS: m/z = 394 [M+H]⁺

### Example 17: 2-(3-Fluoro-3'-methoxy-biphenyl-4-ylcarbamoyl)-3-hydroxy-cyclopent-1-enecarboxylic acid LC/(+)-ESI-MS: m/z = 372 [M+H]⁺

### Example 18:2-(3-Fluoro-3'-methoxy-biphenyl-4-ylcarbamoyl)-5-hydroxy-cyclopent-l-enecarboxylic acid LC/(+)-ESI-MS: m/z = 372 [M+H]⁺

### 3. Inhibition Assay of DHODH activity

The standard assay mixture contained 50 µM decyclo ubichinone, 100 µM dihydroorotate, 60 µM 2,6-dichloroindophenol, as well as 20 mU DHODH The volume activity of the recombinant enzyme used was 30 U/ml. Measurements were conducted in 50 mM TrisHCl (150 mM KCI, 0,1% Triton X-100, pH 8,0) at 30°C in a final volume of 1 ml. The components were mixed, and the reaction was started by adding dihydroorotate. The course of reaction was followed by spectrophotometrically measuring the decrease in absorption at 600 nm for 2 min.

Inhibitory studies were conducted in a standard assay with additional variable amounts of inhibitor. For the determination of the IC₅₀ values (concentration of inhibitor required for 50% inhibition) at least five different inhibitor concentrations were applied.

These investigations were carried out with recombinant human as well as with recombinant murine DHODH provided by Prof. M. Löffler, Marburg, Germany [M. Löffler, Chem. Biol. Interact. 124, (2000), 61-76].

Examples 5, 6, 7, 8, 11, 12, 13, 14, 15, 16, 17 and 18 showed IC₅₀-values (human DHODH) of < 1 µM. Examples 9 and 10 showed IC₅₀-values (human DHODH) of < 5 µM_{.} Examples 1,2, 3 and 4 showed IC₅₀-values (human DHODH) of > 5 µM.

## Claims

1. A compound of the general formula (I) and salts thereof, 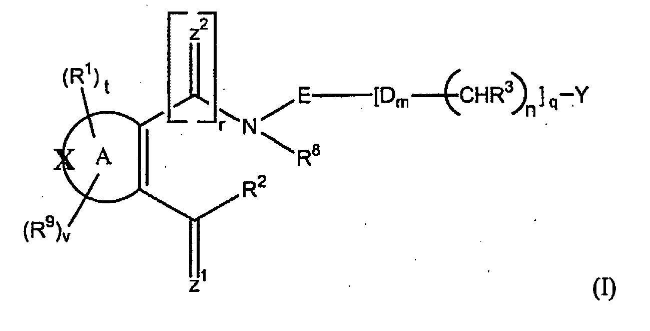 wherein
A is 1-cyclopentene-1,2-diyl;
D is O, S, SO₂, NR⁴ or CH₂;
Z¹ and Z² are independent from each other O, S, or NR⁵
R¹ is independently -CO₂R", -SO₃H, -CONR*R", -CR"O, -SO₂-NR*R", -NO₂, - SO₂-R", -SO-R*, -CN, alkoxy, -OH, -SH, alkylthio, -NR"-CO₂-R', -NR"-CO- R*, -NR"-SO₂-R', -O-CO-R*, -O-CO₂-R*, -O-CO-NR*R"; cycloalkyl, alkylamino, hydroxyalkylamino, aryl which may be substituted by one or more substituents R', or heteroaryl which may be substituted by one or more substituents R';
R⁹ is independently H, halogen, haloalkyl, haloalkyloxy or alkyl;
R* is independently H, alkyl, cycloalkyl, aminoalkyl, alkoxy, -OH, -SH, alkylthio, hydroxyalkyl, haloalkyl, haloalkyloxy, aryl which may be substituted by one or more substituents R' or heteroaryl which may be substituted by one or more substituents R';
R" is independently hydrogen, haloalkyl, hydroxyalkyl, alkyl, cycloalkyl, aryl which may be substituted by one or more substituents R', heteroaryl which may be substituted by one or more substituents R' or aminoalkyl;
R² is H, OR⁶, or NHR⁷;
R³ is H, alkyl, cycloalkyl, aryl which may be substituted by one or more substituents R', arylalkyl, alkoxy, O-aryl, O-cycloalkyl, halogen, aminoalkyl, alkylamino, hydroxylamino, hydroxylalkyl, haloalkyl, haloalkyloxy, heteroaryl which may be substituted by one or more substituents R', alkylthio, S-aryl, or S- cycloalkyl,;
R⁴ is H, alkyl, cycloalkyl, aryl which may be substituted by one or more substituents R', or heteroaryl which may be substituted by one or more substituents R';
R⁵ is H, OH, alkoxy, O-aryl, alkyl, or aryl which may be substituted by one or more substituents R';
R⁶ is H, alkyl, cycloalkyl, aryl which may, be substituted by one or more substituents R'; heteroaryl which may be substituted by one or more substituents R', arylalkyl, alkylaryl, alkoxyakyl, acylmethyl, (acyloxy)alkyl, non- symmetrical (acyloxy)alkyldiester, or dialkylphosphate;
R⁷ is H, alkyl, aryl which may be substituted by one or more substituents R', alkoxy, O-aryl, cycloalkyl, or O-cycloalkyl;
R⁸ is hydrogen or alkyl;
E is a substituted or unsubstituted phenyl ring system;
Y is a substituted or unsubstituted phenyl ring system;
m is 0 or 1;
n is 0 or 1;
p is 0 or 1;
r is 0 or 1;
q is 0 or 1;
t is 1 to 3; and
v is 0 to 3;
R' independently represents H, -CO₂R", -CONHR", -CR"O, -SO₂NR", -NR"- CO-haloalkyl, -NO₂, -NR"-SO₂-haloalkyl, -NR"-SO₂-alkyl, -SO₂-alkyl, -NR"- CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;
an alkyl group denotes a linear or branched C₁-C₆ alkyl group, a linear or branched C₁-C₆ alkenyl group or a linear or branched C₁₋C₆ alkynyl group;
an aryl group denotes an aromatic group having 5 to 15 carbon atoms;
a heteroaryl group denotes a 5- or 6-membered heterocyclic group which contains at least one heteroatom selected from O, N, S and which may be fused to another ring;
a cycloalkyl group denotes a non-aromatic ring system containing 3 to 8 carbon atoms, wherein one or more of the carbon atoms in the ring may be substituted by a group X, X is selected from the group consisting of S, O, N, NR⁴, SO, CO or SO₂.

2. A compound of the general formula (Ia) and salts thereof, 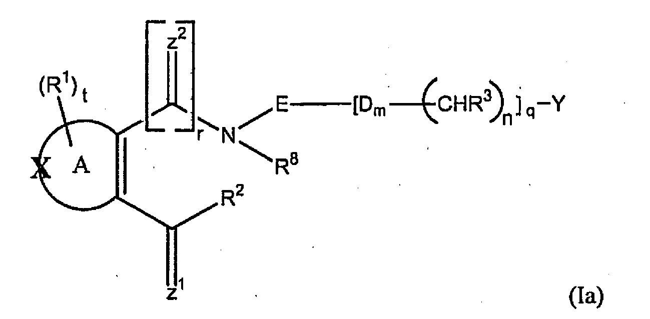 wherein
A is 1-cyclopentene-1,2-diyl;
D is O, S, SO₂, NR⁴ or CH₂;
Z¹ and Z² are independent from each other O, S, or NR⁵;
R¹ is independently H, halogen, haloalkyl, haloalkyloxy -CO₂R", -SO₃H, -OH, - CONR*R", -CR"O, -SO₂-NR*R", -NO₂, -SO₂-R", -SO-R*, -CN, alkoxy, alkylthio, aryl which may be substituted by one or more substituents R', -NR"- CO₂-R', -NR"-CO-R*, -NR"-SO₂-R', -O-CO-R*, -O-CO₂-R*, -O-CO-NR*R"; cycloalkyl, alkylamino, hydroxyalkylamino, -SH, heteroaryl which may be substituted by one or more substituents R', or alkyl;
R* is a group which is independently selected from H, alkyl, cycloalkyl, aminoalkyl, alkoxy, -OH, -SH, alkylthio, hydroxyalkyl, haloalkyl, haloalkyloxy, aryl which may be substituted by one or more substituents R' or heteroaryl which may be substituted by one or more substituents R';
R" is independently hydrogen, haloalkyl, hydroxyalkyl, alkyl, cylcoalkyl, aryl which may be substituted by one or more substituents R', heteroaryl which may be substituted by one or more substituents R' or aminoalkyl;
R² is NHOH or R² together with the nitrogen atom which is attached to R⁸ form a 5 or 6 membered heterocyclic ring with the proviso that R² is -[CH₂]ₛ and R⁸ is absent;
R³ is H, alkyl, cycloalkyl, aryl which may be substituted by one or more substituents R', alkoxy, O-aryl; O-cycloalkyl, halogen, aminoalkyl, alkylamino, hydroxylamino, hydroxylalkyl, haloalkyloxy, heteroaryl which may be substituted by one or more substituents R', alkylthio, S-aryl, S-cycloalkyl, arylalkyl, or haloalkyl,;
R⁴ is H, alkyl, cycloalkyl, aryl which may be substituted by one or more substituents R' or heteroaryl which may be substituted by one or more substituents R';
R⁵ is H, OH, alkoxy, O-aryl, alkyl or aryl which may be substituted by one or more substituents R';
R⁸ is hydrogen, or alkyl;
E is a substituted or unsubstituted phenyl ring system;
Y is a substituted or unsubstituted phenyl ring system;
m is 0 or 1;
n is 0 or 1;
p is 0 or 1;
r is 0 or 1;
q is 0 or 1;
s is 0 to 2; and
t is 0 to 3;
R' independently represents H, -CO₂R", -CONHR", -CR"O, -SO₂NR", -NR"- CO-haloalkyl, -NO₂, -NR"-SO₂-haloalkyl, -NR"-SO₂-aLkyL, -SO₂-alkyl, -NR"- CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;
an alkyl group denotes a linear or branched C₁-C₆ alkyl group, a linear or branched C₁-C₆ alkenyl group or a linear or branched C₁-C₆ alkynyl group;
an aryl group denotes an aromatic group having 5 to 15 carbon atoms;
a heteroaryl group denotes a 5- or 6-membered heterocyclic group which contains at least one heteroatom selected from O, N, S and which may be fused to another ring;
a cycloalkyl group denotes a non-aromatic ring system containing 3 to 8 carbon atoms, wherein one or more of the carbon atoms in the ring may be substituted by a group X, X is selected from the group consisting of S, O, N, NR⁴, SO, CO or SO₂.

3. A pharmaceutical composition comprising a compound as defined in claim 1 or 2 in free form or in the form of a pharmaceutically acceptable salt and a pharmaceutically acceptable diluent or carrier.

4. A compound according to claim 1 for the use as a medicament.

5. A compound according to claim 2 for the use as a medicament.

6. The use of a compound as defined in claim 1 or a pharmacologically tolerable salt thereof in the manufacture of a medicament for use in treatment of a disease or a therapeutic indication selected from the group comprising fibrosis, uveitis, rhinitis, asthma or arthropathy, in particular arthrosis, all forms of rheumatism; acute immunological events and disorders such as sepsis, septic shock, endotoxic shock, Gram-negative sepsis, toxic shock syndrome, acute respiratory distress syndrome, stroke, reperfusion injury, CNS injury, serious forms of allergy, graft versus host and host versus graft reactions, alzheimer's or pyresis, restenosis, chronic pulmonary inflammatory disease, silicosis, pulmonary sarcosis, bone resorption disease; these immunological events also include a desired modulation and suppression of the immune system; all types of autoimmune disease, in particular rheumatoid arthritis, rheumatoid spondylitis, osteoarthritis, gouty arthritis, multiple sclerosis, insulin dependent diabetes mellitus and non-insulin dependent diabetes, and lupus erythematoidis, ulcerative colitis, Morbus Crohn, inflammatory bowel disease, as well as other chronic inflammations, chronic diarrhea; dermatological disorders such as psoriasis, progressive retinal atrophy, all kinds of infections including opportunistic infections.

7. The use of a compound as defined in claim 2 or pharmacologically tolerable salt thereof in the manufacture of a medicament for use in treatment of a disease or a therapeutic indication selected from the group comprising fibrosis, uveitis, rhinitis, asthma or arthropathy, in particular arthrosis, all forms of rheumatism; acute immunological events and disorders such as sepsis, septic shock, endotoxic shock, Gram-negative sepsis, toxic shock syndrome, acute respiratory distress syndrome, stroke, reperfusion injury, CNS injury, serious forms of allergy, graft versus host and host versus graft reactions, alzheimer's or pyresis, restenosis, chronic pulmonary inflammatory disease, silicosis, pulmonary sarcosis, bone resorption disease; these immunological events also include a desired modulation and suppression of the immune system; all types of autoimmune disease, in particular rheumatoid arthritis, rheumatoid spondylitis, osteoarthritis, gouty arthritis, multiple sclerosis, insulin dependent diabetes mellitus and non-insulin dependent diabetes, and lupus erythematoidis, ulcerative colitis, Morbus Crohn, inflammatory bowel disease, as well as other chronic inflammations, chronic diarrhea; dermatological disorders such as psoriasis, progressive retinal atrophy, all kinds of infections including opportunistic infections.

8. The use of a compound as defined in claim 6 or 7 wherein the disease or indication is selected, from the group consisting of rheumatism, acute immunological disorders, autoimmune diseases, diseases caused by malignant cell proliferation, inflammatory diseases, diseases that are caused by protozoal infestations in humans and animals, diseases that are caused by viral infections and *Pneumocycstis carinii,* fibrosis, uveitis, rhinitis, asthma and athropathy.

9. The use of a compound as defined in claim 1 or 2 for the *in vitro* inhibition of DHODH.

10. Pharmaceutical composition for use in treating or preventing the disease or indication selected from the group comprising fibrosis, uveitis, rhinitis, asthma or arthropathy, in particular arthrosis, all forms of rheumatism; acute immunological events and disorders such as sepsis, septic shock, endotoxic shock, Gram-negative sepsis, toxic shock syndrome, acute respiratory distress syndrome, stroke, reperfusion injury, CNS injury, serious forms of allergy, graft versus host and host versus graft reactions, alzheimer's or pyresis, restenosis, chronic pulmonary inflammatory disease, silicosis, pulmonary sarcosis, bone resorption disease; these immunological events also include a desired modulation and suppression of the immune system; all types of autoimmune disease, in particular rheumatoid arthritis, rheumatoid spondylitis, osteoarthritis, gouty arthritis, multiple sclerosis, insulin dependent diabetes mellitus and non-insulin dependent diabetes, and lupus erythematoidis, ulcerative colitis, Morbus Crohn, inflammatory bowel disease, as well as other chronic inflammations, chronic diarrhea; dermatological disorders such as psoriasis, progressive retinal atrophy, all kinds of infections including opportunistic infections, comprising compounds as defined in claim 1 or 2.

11. Pharmaceutical composition for use in treating or preventing the disease or indication selected from the group consisting of rheumatism, acute immunological disorders, autoimmune diseases, diseases caused by malignant cell proliferation, inflammatory diseases, diseases that are caused by protozoal infestations in humans and animals, diseases that are caused by viral infections and *Pneumocycstis carinii,* fibrosis, uveitis, rhinitis, asthma and athropathy comprising compounds as defined in claim 1 or 2.

## Patentansprüche

1. Verbindung der allgemeinen Formel (I) und Salze davon, 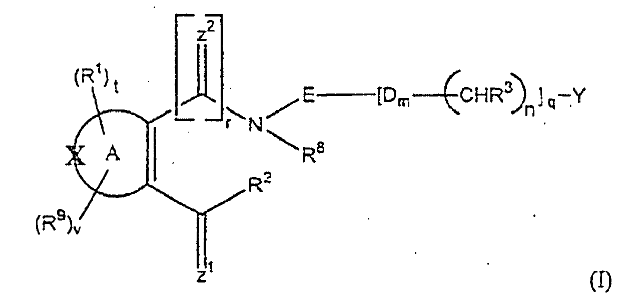 wobei
A 1-Cyclopenten-1,2-diyl ist,
D O, S, SO₂, NR⁴ oder CH₂ ist;
Z¹ und Z² unabhängig voneinander O, S oder NR⁵ sind;
R¹ unabhängig -CO₂R", -SO₃H, -CONR*R", -CR"O, -SO₂-NR*R", -NO₂, -SO₂- R", -SO-R*, -CN, Alkoxy, -OH, -SH, Alkylthio, NR"-CO₂-R', -NR"-CO- R*, -NR"-SO₂-R', -O-CO-R*, -O-CO₂-R*, -O-CO-NR*R"; Cycloalkyl, Alkylamino, Hydroxyalkylamino, Aryl, das mit einem oder mehreren Substituenten R' substituiert sein kann, oder Heteroaryl, das mit einem oder mehreren Substituenten R' substituiert sein kann, ist;
R⁹ unabhängig H, Halogen, Halogenalkyl, Halogenalkyloxy oder Alkyl ist;
R* unabhängig H, Alkyl, Cycloalkyl, Aminoalkyl, Alkoxy, -OH, -SH, Alkylthio, Hydroxyalkyl, Halogenalkyl, Halogenalkyloxy, Aryl, das mit einem oder mehreren Substituenten R' substituiert sein kann, oder Heteroaryl, das mit einem oder mehreren Substituenten R' substituiert sein kann, ist;
R" unabhängig Wasserstoff, Halogenalkyl, Hydroxyalkyl, Alkyl, Cycloalkyl, Aryl, das mit einem oder mehreren Substituenten R' substituiert sein kann, Heteroaryl, das mit einem oder mehreren Substituenten R' substituiert sein kann, oder Aminoalkyl ist;
R² H, OR⁶ oder NHR⁷ ist;
R³ H, Alkyl, Cycloalkyl, Aryl, das mit einem oder mehreren Substituenten R' substituiert sein kann, Arylalkyl, Alkoxy, O-Aryl, O-Cycloalkyl, Halogen, Aminoalkyl, Alkylamino, Hydroxylamino, Hydroxylalkyl, Halogenalkyl, Halogenalkyloxy, Heteroaryl, das mit einem oder mehreren Substituenten R' substituiert sein kann, Alkylthio, S-Aryl oder S-Cycloalkyl ist;
R⁴ H, Alkyl, Cycloalkyl, Aryl, das mit einem oder mehreren Substituenten R' substituiert sein kann, oder Heteroaryl, das mit einem oder mehreren Substituenten R' substituiert sein kann, ist;
R⁵ H, OH, Alkoxy, O-Aryl, Alkyl oder Aryl, das mit einem oder mehreren Substituenten R' substituiert sein kann, ist;
R⁶ H, Alkyl, Cycloalkyl, Aryl, das mit einem oder mehreren Substituenten R' substituiert sein kann; Heteroaryl, das mit einem oder mehreren Substituenten R' substituiert sein kann, Arylalkyl, Alkylaryl, Alkoxyalkyl, Acylmethyl, (Acyloxy)alkyl, nicht-symmetrischer (Acyloxy)alkyldiester oder Dialkylphosphat ist;
R⁷ H, Alkyl, Aryl, das mit einem oder mehreren Substituenten R' substituiert sein kann, Alkoxy, O-Aryl, Cycloalkyl oder O-Cycloalkyl ist;
R⁸ Wasserstoff oder Alkyl ist;
E ein substituiertes oder unsubstituiertes Phenylringsystem ist;
Y ein substituiertes oder unsubstituiertes Phenylringsystem ist;
m 0 oder 1 ist;
n 0 oder 1 ist;
p 0 oder 1 ist;
r 0 oder 1 ist;
q 0 oder 1 ist;
t 1 bis 3 ist; und
v 0 bis 3 ist;
R' unabhängig H, -CO₂R", -CONHR", -CR"O, -SO₂NR", -NR"-CO- Halogenalkyl, NO₂, -NR"-SO₂-Halogenalkyl, -NR"-SO₂-Alkyl, -SO₂-Alkyl, -NR"-CO-Alkyl, -CN, Alkyl, Cycloalkyl, Aminoalkyl, Alkylamino, Alkoxy, -OH, -SH, Alkylthio, Hydroxyalkyl, Hydroxyalkylamino, Halogen, Halogenalkyl, Halogenalkyloxy, Aryl, Arylalkyl oder Heteroaryl ist;
ein Alkylrest einen linearen oder verzweigten C₁-C₆-Alkylrest, einen linearen oder verzweigten C₁-C₆-Alkenylrest oder einen linearen oder verzweigten C₁-C₆-Alkinylrest bezeichnet;
ein Arylrest einen aromatischen Rest mit 5 bis 15 Kohlenstoffatomen bezeichnet;
ein Heteroarylrest einen 5- oder 6-gliedrigen heterocyclischen Rest bezeichnet, der mindestens ein Heteroatom, ausgewählt aus O, N, S, enthält und an einen anderen Ring kondensiert sein kann;
ein Cycloalkylrest ein nicht-aromatisches Ringsystem bezeichnet, das 3 bis 8 Kohlenstoffatome enthält, wobei eines oder mehrere der Kohlenstoffatome in dem Ring mit einem Rest X substituiert sein können, wobei X aus der Gruppe bestehend aus S, O, N, NR⁴, SO, CO oder SO₂ ausgewählt ist.

2. Verbindung der allgemeinen Formel (Ia) und Salze davon, 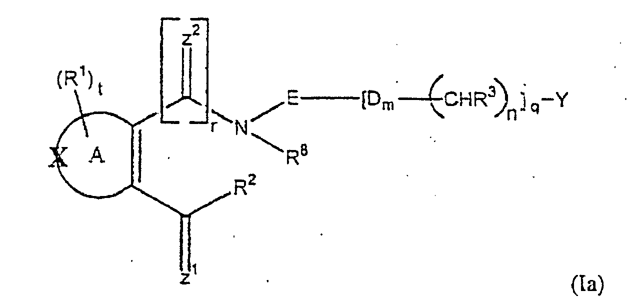 wobei
A 1-Cyclopenten-1,2-diyl ist;
D O, S, SO₂, NR⁴ oder CH₂ ist;
Z¹ und Z² unabhängig voneinander O, S oder NR⁵ sind;
R¹ unabhängig H, Halogen, Halogenalkyl, Halogenalkyloxy, -CO₂R", -SO₃H, -OH, -CONR*R", -CR"O, -SO₂-NR*R", -NO₂, -SO₂-R", -SO-R*, -CN, Alkoxy, Alkylthio, Aryl, das mit einem oder mehreren Substituenten R' substituiert sein kann, NR"-CO₂-R', NR"-CO-R*, -NR"-SO₂₋R', -O-CO-R*, -O-CO₂-R*, -O-CO-NR*R"; Cycloalkyl, Alkylamino, Hydroxyalkylamino, -SH, Heteroaryl, das mit einem oder mehreren Substituenten R' substituiert sein kann, oder Alkyl ist;
R* ein Rest ist, der unabhängig aus H, Alkyl, Cycloalkyl, Aminoalkyl, Alkoxy, -OH, -SH, Alkylthio, Hydroxyalkyl, Halogenalkyl, Halogenalkyloxy, Aryl, das mit einem oder mehreren Substituenten R' substituiert sein kann, oder Heteroaryl, das mit einem oder mehreren Substituenten R' substituiert sein kann, ausgewählt ist;
R" unabhängig Wasserstoff, Halogenalkyl, Hydroxyalkyl, Alkyl, Cycloalkyl, Aryl, das mit einem oder mehreren Substituenten R' substituiert sein kann, Heteroaryl, das mit einem oder mehreren Substituenten R' substituiert sein kann, oder Aminoalkyl ist;
R² NHOH ist oder R² zusammen mit dem Stickstoffatom, das an R⁸ gebunden ist, einen 5- oder 6-gliedrigen heterocyclischen Ring bildet, mit der Maßgabe, dass R² -[CH₂]₅ ist und R⁸ abwesend ist;
R³ H, Alkyl, Cycloalkyl, Aryl, das mit einem oder mehreren Substituenten R' substituiert sein kann, Alkoxy, O-Aryl, O-Cycloalkyl, Halogen, Aminoalkyl, Alkylamino, Hydroxylamino, Hydroxyalkyl, Halogenalkyloxy, Heteroaryl, das mit einem oder mehreren Substituenten R' substituiert sein kann, Alkylthio, S-Aryl, S- Cycloalkyl, Arylalkyl oder Halogenalkyl ist;
R⁴ H, Alkyl, Cycloalkyl, Aryl, das mit einem oder mehreren Substituenten R' substituiert sein kann, oder Heteroaryl, das mit einem oder mehreren Substituenten R' substituiert sein kann, ist;
R⁵ H, OH, Alkoxy, O-Aryl, Alkyl oder Aryl, das mit einem oder mehreren Substituenten R' substituiert sein kann, ist;
R⁸ Wasserstoff oder Alkyl ist;
E ein substituiertes oder unsubstituiertes Phenylringsystem ist;
Y ein substituiertes oder unsubstituiertes Phenylringsystem ist;
m 0 oder 1 ist;
n 0 oder 1 ist;
p 0 oder 1 ist;
r 0 oder 1 ist;
q 0 oder 1 ist;
s 0 bis 2 ist; und
t 0 bis 3 ist;
R' unabhängig H, -CO₂R", -CONHR", -CR"O, -SO₂NR", NR"-CO- Halogenalkyl, -NO₂, -NR"-SO₂-Halogenalkyl, -NR"-SO₂-Alkyl, -SO₂-Alkyl, -NR"-CO-Alkyl, -CN, Alkyl, Cycloalkyl, Aminoalkyl, Alkylamino, Alkoxy, -OH, -SH, Alkylthio, Hydroxyalkyl, Hydroxyalkylamino, Halogen, Halogenalkyl, Halogenalkyloxy, Aryl, Arylalkyl oder Heteroaryl bedeutet;
ein Alkylrest einen linearen oder verzweigten C₁-C₆-Alkylrest, einen linearen oder verzweigten C₁-C₆-Alkenylrest oder einen linearen oder verzweigten C₁-C₆-Alkinylrest bezeichnet;
ein Arylrest einen aromatischen Rest mit 5 bis 15 Kohlenstoffatomen bezeichnet;
ein Heteroarylrest einen 5- oder 6-gliedrigen heterocyclischen Rest bezeichnet, der mindestens ein Heteroatom, ausgewählt aus O, N, S, enthält und an einen anderen Ring kondensiert sein kann;
ein Cycloalkylrest ein nicht-aromatisches Ringsystem bezeichnet, das 3 bis 8 Kohlenstoffatome enthält, wobei eines oder mehrere der Kohlenstoffatome in dem Ring mit einem Rest X substituiert sein können, wobei X aus der Gruppe bestehend aus S, O, N, NR⁴, SO, CO oder SO₂ ausgewählt ist.

3. Arzneimittel, umfassend eine wie in Anspruch 1 oder 2 definierte Verbindung in freier Form oder in der Form eines pharmazeutisch verträglichen Salzes und ein pharmazeutisch verträgliches Verdünnungsmittel oder ein pharmazeutisch verträglicher Träger.

4. Verbindung nach Anspruch 1 zur Verwendung als Medikament.

5. Verbindung nach Anspruch 2 zur Verwendung als Medikament.

6. Verwendung einer wie in Anspruch 1 definierten Verbindung oder eines pharmakologisch verträglichen Salzes davon bei der Herstellung eines Medikaments zur Verwendung bei der Behandlung einer Erkrankung oder einer therapeutischen Indikation, ausgewählt aus der Gruppe umfassend Fibrose, Uveitis, Rhinitis, Asthma oder Arthropathie, insbesondere Arthrose, alle Formen von Rheumatismus; akute immunologische Vorfälle und Störungen, wie z.B. Sepsis, septischer Schock, endotoxischer Schock, Gram-negative Sepsis, toxisches Schocksyndrom, akutes Atemnotsyndrom, Schlaganfall, Reperfusionsschädigung, ZNS-Schädigung, schwere Formen von Allergie, Transplantat-gegen-Wirt- und Wirt-gegen-Transplantat-Reaktionen, Alzheimer oder Pyrosis, Restenose, chronische Lungenentzündung, Silicose, Lungensarkose, Knochenresorptionskrankheit; diese immunologischen Vorfälle beinhalten auch eine gewünschte Modulation und Unterdrückung des Immunsystems; alle Arten von Autoimmunerkrankungen, inbesondere rheumatoide Arthritis, rheumatoide Spondylitis, Osteoarthritis, Gichtarthritis, Multiple Sklerose, insulinabhängigen Diabetes mellitus und nicht-insulinabhängigen Diabetes, und Lupus erythematodes, Colitis ulcerosa, Morbus Crohn, entzündliche Darmerkrankung, sowie andere chronische Entzündungen, chronischen Durchfall; dermatologische Störungen, wie z.B. Psoriasis, progressive Retinaatrophie, alle Arten von Infektionen einschließlich opportunistischer Infektionen.

7. Verwendung einer wie in Anspruch 2 definierten Verbindung oder eines pharmakologisch vertäglichen Salzes davon bei der Herstellung eines Medikaments zur Verwendung bei der Behandlung einer Erkrankung oder einer therapeutischen Indikation, ausgewählt aus der Gruppe umfassend Fibrose, Uveitis, Rhinitis, Asthma oder Arthropathie, insbesondere Arthrose, alle Formen von Rheumatismus; akute immunologische Vorfälle und Störungen, wie z.B. Sepsis, septischer Schock, endotoxischer Schock, Gram-negative Sepsis, toxisches Schocksyndrom, akutes Atemnotsyndrom, Schlaganfall, Reperfusionsschädigung, ZNS-Schädigung, schwere Formen von Allergie, Transplantat-gegen-Wirt- und Wirt-gegen-Transplantat-Reaktionen, Alzheimer oder Pyrosis, Restenose, chronische Lungenentzündung, Silicose, Lungensarkose, Knochenresorptionskrankheit; diese immunologischen Vorfälle beinhalten auch eine gewünschte Modulation und Unterdrückung des Immunsystems; alle Arten von Autoimmunerkrankungen, inbesondere rheumatoide Arthritis, rheumatoide Spondylitis, Osteoarthritis, Gichtarthritis, Multiple Sklerose, insulinabhängigen Diabetes mellitus und nicht-insulinabhängigen Diabetes, und Lupus erythematodes, Colitis ulcerosa, Morbus Crohn, entzündliche Darmerkrankung, sowie andere chronische Entzündungen, chronischen Durchfall; dermatologische Störungen, wie z.B. Psoriasis, progressive Retinaatrophie, alle Arten von Infektionen einschließlich opportunistischer Infektionen.

8. Verwendung einer wie in Anspruch 6 oder 7 definierten Verbindung, wobei die Erkrankung oder Indikation aus der Gruppe bestehend aus Rheumatismus, akuten immunologischen Störungen, Autoimmunerkrankungen, durch bösartiges Zellwachstum verursachten Erkrankungen, entzündlichen Erkrankungen, Erkrankungen, die durch Protozoenbefall bei Mensch und Tier ausgelöst werden, Erkrankungen, die durch Virusinfektionen und Pneumocycstis carinii ausgelöst werden, Fibrose, Uveitis, Rhinitis, Asthma und Arthropathie ausgewählt ist.

9. Verwendung einer wie in Anspruch 1 oder 2 definierten Verbindung zur *in vitro-*Hemmung von DHODH.

10. Arzneimittel zur Verwendung bei der Behandlung oder Prävention der aus der Gruppe bestehend aus Fibrose, Uveitis, Rhinitis, Asthma oder Arthropathie, insbesondere Arthrose, allen Formen von Rheumatismus; akuten immunologischen Vorfällen und Störungen, wie z.B. Sepsis, septischem Schock, endotoxischem Schock, Gram-negativer Sepsis, toxischem Schocksyndrom, akutem Atemnotsyndrom, Schlaganfall, Reperfusionsschädigung, ZNS-Schädigung, schweren Formen von Allergie, Transplantat-gegen-Wirt- und Wirt-gegen-Transplantat-Reaktionen, Alzheimer oder Pyrosis, Restenose, chronischer Lungenentzündung, Silicose, Lungensarkose, Knochenresorptionskrankheit; diese immunologischen Vorfälle beinhalten auch eine gewünschte Modulation und Unterdrückung des Immunsystems; allen Arten von Autoimmunerkrankungen, inbesondere rheumatoider Arthritis, rheumatoider Spondylitis, Osteoarthritis, Gichtarthritis, Multipler Sklerose, insulinabhängigem Diabetes mellitus und nicht-insulinabhängigem Diabetes, und Lupus erythematodes, Colitis ulcerosa, Morbus Crohn, entzündlichen Darmerkrankungen, sowie anderen chronischen Entzündungen, chronischem Durchfall; dermatologischen Störungen, wie z.B. Psoriasis, progressiver Retinaatrophie, allen Arten von Infektionen einschließlich opportunistischer Infektionen ausgewählten Erkrankung oder Indikation, umfassend wie in Anspruch 1 oder 2 definierte Verbindungen.

11. Arzneimittel zur Verwendung bei der Behandlung oder Prävention der aus der Gruppe bestehend aus Rheumatismus, akuten immunologischen Störungen, Autoimmunerkrankungen, durch bösartiges Zellwachstum verursachten Erkrankungen, entzündlichen Erkrankungen, Erkrankungen, die durch Protozoenbefall bei Mensch und Tier ausgelöst werden, Erkrankungen, die durch Virusinfektionen und *Pneumocycstis carinii* ausgelöst werden, Fibrose, Uveitis, Rhinitis, Asthma und Arthropathie ausgewählten Erkrankung oder Indikation, umfassend wie in Anspruch 1 oder 2 definierte Verbindungen.

## Revendications

1. Composés de formule générale (I) et ses sels, 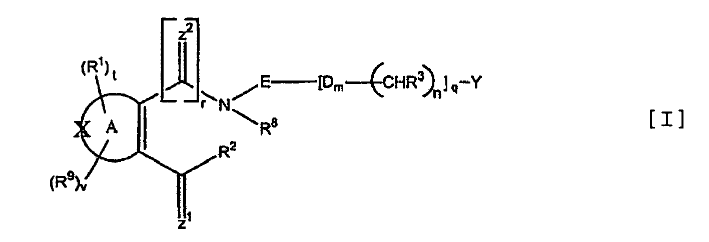 où
A est 1-cyclopentène-1,2-diyle ;
D est O, S, SO₂, NR⁴ ou CH₂ ;
Z¹ et Z² sont indépendamment l'un de l'autre O, S ou NR⁵ ;
R¹ est indépendamment -CO₂R", -SO₃H, -CONR*R", -CR"O, -SO₂-NR*R", -NO₂, -SO₂-R", -SO-R*, -CN, alcoxy, -OH, -SH, alkylthio, -NR"-CO₂-R', -NR"-CO-R*, -NR''-SO₂-R', -O-CO-R*, -O-CO₂-R*, -O-CO-NR*R'' ; cycloalkyle, alkylamino, hydroxyalkylamino, aryle qui peut être substitué par un ou plusieurs substituants R', ou hétéroaryle qui peut être substitué par un ou plusieurs substituants R' ;
R⁹ est indépendamment H, halogène, halogénoalkyle, halogénoalkyloxy ou alkyle ;
R* est indépendamment H, alkyle, cycloalkyle, aminoalkyle, alcoxy, -OH, -SH, alkylthio, hydroxyalkyle, halogénoalkyle, halogénoalkyloxy, aryle qui peut être substitué par un ou plusieurs substituants R' ou hétéroaryle qui peut être substitué par un ou plusieurs substituants R' ;
R" est indépendamment hydrogène, halogénoalkyle, hydroxyalkyle, alkyle, cycloalkyle, aryle qui peut être substitué par un ou plusieurs substituants R', hétéroaryle qui peut être substitué par un ou plusieurs substituants R' ou aminoalkyle ;
R² est H, OR⁶ ou NHR⁷ ;
R³ est H, alkyle, cycloalkyle, aryle qui peut être substitué par un ou plusieurs substituants R', arylalkyle, alcoxy, O-aryle, O-cycloalkyle, halogène, aminoalkyle, alkylamino, hydroxylamino, hydroxylalkyle, halogénoalkyle, halogénoalkyloxy, hétéroaryle qui peut être substitué par un ou plusieurs substituants R', alkylthio, S-aryle ou S-cycloalkyle ;
R⁴ est H, alkyle, cycloalkyle, aryle qui peut être substitué par un ou plusieurs substituants R' ou hétéroaryle qui peut être substitué par un ou plusieurs substituants R' ;
R⁵ est H, OH, alcoxy, O-aryle, alkyle ou aryle qui peut être substitué par un ou plusieurs substituants R' ;
R⁶ est H, alkyle, cycloalkyle, aryle qui peut être substitué par un ou plusieurs substituants R' ; hétéroaryle qui peut être substitué par un ou plusieurs substituants R' ; arylalkyle, alkylaryle, alcoxyalkyle, acylméthyle, (acyloxy)alkyle, un diester d'(acyloxy)alkyle non-symétrique, ou un phosphate de dialkyle.
R⁷ est H, alkyle, aryle qui peut être substitué par un ou plusieurs substituants R', alcoxy, O-aryle, cycloalkyle ou O-cycloalkyle ;
R⁸ est hydrogène ou alkyle ;
E est un système de cycle phényle substitué ou non substitué ;
Y est un système de cycle phényle substitué ou non substitué ;
m vaut 0 ou 1 ;
n vaut 0 ou 1 ;
p vaut 0 ou 1 ;
r vaut 0 ou 1 ;
q vaut 0 ou 1 ;
t vaut 1 à 3 ; et
v vaut 0 à 3 ;
R' représente indépendamment H, -CO₂R'', -CONHR", -CR''O, -SO₂NR", -NR"-CO-halogénoalkyle, -NO₂, -NR"-SO₂-halogénoalkyle, -NR"-SO₂-alkyle, -SO₂-alkyle, -NR"-CO-alkyle, -CN, alkyle, cycloalkyle, aminoalkyle, alkylamino, alcoxy, -OH, -SH, alkylthio, hydroxyalkyle, hydroxyalkylamino, halogène, halogénoalkyle, halogénoalkyloxy, aryle, arylalkyle ou hétéroaryle ;
un groupe alkyle désigne un groupe alkyle en C₁ à C₆ linéaire ou ramifié, un groupe alcényle en C₁ à C₆ linéaire ou ramifié ou un groupe alcynyle en C₁ à C₆ linéaire ou ramifié ;
un groupe aryle désigne un groupe aromatique comportant 5 à 15 atomes de carbone ;
un groupe hétéroaryle désigne un groupe hétérocyclique à 5 ou 6 éléments qui contient au moins un hétéroatome choisi parmi O, N, S et qui peut être fusionné, avec un autre cycle ;
un groupe cycloalkyle désigne un système de cycle non aromatique contenant 3 à 8 atomes de carbone, dans lequel un ou plusieurs des atomes de carbone sur le cycle peuvent être substitués par un groupe X, X étant choisi dans le groupe constitué par S, O, N, NR⁴, SO, CO ou SO₂.

2. Composé de formule générale (Ia) et ses sels, 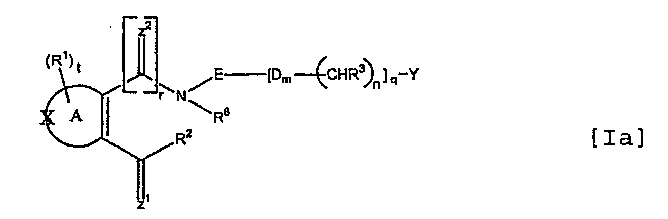 où
A est 1-cyclopentène-1,2-diyle ;
D est O, S, SO₂, NR⁴ ou CH₂ ;
Z¹ et Z² sont indépendamment l'un de l'autre O, S ou NR-⁵ ;
R¹ est indépendamment H, halogène, halogénoalkyle, halogénoalkyloxy, -CO₂R", -SO₃H, -OH, -CONR*R", -CR"O, -SO₂-NR*R", -NO₂, -SO₂-R", -SO-R*, -CN, alcoxy, alkylthio, aryle qui peut être substitué par un ou plusieurs substituants R', -NR"-CO₂-R', -NR"-CO-R*, -NR"-SO₂-R*, -O-CO₂-R*, -O-CO-NR*R"; cycloalkyle, alkylamino, hydroxyalkylamino, -SH, hétéroaryle qui peut être substitué par un ou plusieurs substituants R', ou alkyle ;
R* est un groupe qui est indépendamment choisi parmi H, alkyle, cycloalkyle, aminoalkyle, alcoxy, -OH, -SH, alkylthio, hydroxyalkyle, halogénoalkyle, halogénoalkyloxy, aryle qui peut être substitué par un ou plusieurs substituants R' ou hétéroaryle qui peut être substitué par un ou plusieurs substituants R' ;
R" est indépendamment hydrogène, halogénoalkyle, hydroxyalkyle, alkyle, cycloalkyle, aryle qui peut être substitué par un ou plusieurs substituants R', hétéroaryle qui peut être substitué par un ou plusieurs substituants R' ou aminoalkyle ;
R² est NHOH ou bien R² conjointement avec l'atome d'azote qui est attaché à R⁸ forment un cycle hétérocyclique à 5 à 6 éléments à condition que R² soit -[CH₂]ₛ et R⁸ soit absent ;
R³ est H, alkyle, cycloalkyle, aryle qui peut être substitué par un ou plusieurs substituants R', alcoxy, O-aryle, O-cycloalkyle, halogène, aminoalkyle, alkylamino, hydroxylamino, hydroxylalkyle, halogénoalkyloxy, hétéroaryle qui peut être substitué par un ou plusieurs substituants R', alkylthio, S-aryle, S-cycloalkyle, arylakyle ou halogénoakyle ;
R⁴ est H, alkyle, cycloalkyle, aryle qui peut être substitué par un ou plusieurs substituants R' ou hétéroaryle qui peut être substitué par un ou plusieurs substituants R' ;
R⁵ est H, OH, alcoxy, O-aryle, alkyle ou aryle qui peut être substitué par un ou plusieurs substituants R' ;
R⁸ est hydrogène ou alkyle ;
E est un système de cycle phényle substitué ou non substitué ;
Y est un système de cycle phényle substitué ou non substitué ;
m vaut 0 ou 1 ;
n vaut 0 ou 1 ;
p vaut 0 ou 1 ;
r vaut 0 ou 1 ;
q vaut 0 ou 1 ;
s vaut 0 à 2 ; et
t vaut 0 à 3 ;
R' représente indépendamment H, -CO₂R", -CONHR", -CR"O, -SO₂NR", -NR"-CO-halogénoalkyle, -NO₂, -NR"-SO₂-halogénoalkyle, -NR"-SO₂-alkyle, -SO₂-alkyle, -NR "-CO-alkyle, -CN, alkyle, cycloalkyle, aminoalkyle, alkylamino, alcoxy, -OH, -SH, alkylthio, hydroxyalkyle, hydroxyalkylamino, halogène, halogénoalkyle, halogénoalkyloxy, aryle, arylalkyle ou hétéroaryle ;
un groupe alkyle désigne un groupe alkyle en C₁ à C₆ linéaire ou ramifié, un groupe alcényle en C₁ à C₆ linéaire ou ramifié ou un groupe alcynyle en C₁ à C₆ linéaire ou ramifié ;
un groupe aryle désigne un groupe aromatique comportant 5 à 15 atomes de carbones ;
un groupe hétéroaryle désigne un groupe hétérocyclique à 5 ou 6 éléments qui contient au moins un hétéroatome choisi parmi O, N, S et qui peut être fusionné avec un autre cycle ;
un groupe cycloalkyle désigne un système de cycle non aromatique contenant 3 à 8 atomes de carbone, dans lequel un ou plusieurs des atomes de carbone sur le cycle peuvent être substitués par un groupe X, X étant choisi dans le groupe constitué par S, O, N, NR⁴, SO, CO ou SO₂.

3. Composition pharmaceutique comprenant un composé tel que défini dans la revendication 1 ou 2 sous forme libre ou sous la forme d'un sel pharmaceutiquement acceptable et d'un diluant ou d'un support pharmaceutiquement acceptable.

4. Composé selon la revendication 1 à utiliser comme médicament.

5. Composé selon la revendication 2 à utiliser comme médicament.

6. Utilisation d'un composé tel que défini dans la revendication 1 ou d'un sel pharmacologiquement tolérable de celui-ci dans la fabrication d'un médicament à utiliser dans le traitement d'une maladie ou d'une indication thérapeutique choisie dans le groupe comprenant la fibrose, l'uvéite, la rhinite, l'asthme ou l'arthropathie, en particulier l'arthrose, toutes les formes de rhumatisme ; des événements et troubles immunologiques aigus tels que la sepsie, le choc septique, le choc endotoxique ; la sepsie Gram-négative, le syndrome du choc toxique, le syndrome de détresse respiratoire aigu, l'accident vasculaire cérébral, la lésion de reperfusion, la lésion du système nerveux central, des formes sérieuses d'allergie, des réactions greffe contre hôte et hôte contre greffe, la maladie d'Alzheimer ou la pyrésie, la resténose, une maladie inflammatoire pulmonaire chronique, la silicose, la sarcose pulmonaire, une maladie de résorption osseuse ; ces événements immunologiques comprennent également une modulation et une suppression souhaitées du système immunitaire ; tous les types de maladies auto-immunes, notamment la polyarthrite rhumatoïde, la spondylite rhumatoïde, l'arthrose, l'arthrite goutteuse, la sclérose en plaques, le diabète sucré insulinodépendant et le diabète non insulinodépendant, et le lupus érythémateux, la rectocolite hémorragique, la maladie de Morbus Crohn, une infection abdominale inflammatoire, ainsi que d'autres inflammations chroniques, la diarrhée chronique ; les troubles dermatologiques tels que le psoriasis, l'atrophie rétinienne progressive, tous les types d'infections, y compris les infections opportunistes.

7. Utilisation d'un composé tel que défini dans la revendication 2 ou d'un sel pharmacologiquement tolérable de celui-ci dans la fabrication d'un médicament à utiliser dans le traitement d'une maladie ou d'une indication thérapeutique choisie dans le groupe comprenant la fibrose, l'uvéite, la rhinite, l'asthme ou l'arthropathie, en particulier l'arthrose, toutes les formes de rhumatisme ; des événements et troubles immunologiques aigus tels que la sepsie, le choc septique, le choc endotoxique ; la sepsie Gram-négative, le syndrome du choc toxique, le syndrome de détresse respiratoire aigu, l'accident vasculaire cérébral, la lésion de reperfusion, la lésion du système nerveux central, des formes sérieuses d'allergie, des réactions greffe contre hôte et hôte contre greffe, la maladie d'Alzheimer ou la pyrésie, la resténose, une maladie inflammatoire pulmonaire chronique, la silicose, la sarcose pulmonaire, une maladie de résorption osseuse ; ces événements immunologiques comprennent également une modulation et une suppression souhaitées du système immunitaire ; tous les types de maladies auto-immunes, notamment la polyarthrite rhumatoïde, la spondylite rhumatoïde, l'arthrose, l'arthrite goutteuse, la sclérose en plaques, le diabète sucré insulinodépendant et le diabète non insulinodépendant, et le lupus érythémateux, la rectocolite hémorragique, la maladie de Morbus Crohn, une infection abdominale inflammatoire, ainsi que d'autres inflammations chroniques, la diarrhée chronique ; les troubles dermatologiques tels que le psoriasis, l'atrophie rétinienne progressive, tous les types d'infections, y compris les infections opportunistes.

8. Utilisation d'un composé tel que défini dans la revendication 6 ou 7, dans laquelle la maladie ou l'indication est choisie dans le groupe constitué par un rhumatisme, les troubles immunologiques aigus, les maladies auto-immunes, les maladies provoquées par une prolifération de cellules malignes, les maladies inflammatoires, les maladies provoquées par des infestations protozoaires chez l'être humain et l'animal, les maladies provoquées par des infections virales et *Pneumocycstis carinii,* la fibrose, l'uvéite, la rhinite, l'asthme et l'arthropathie.

9. Utilisation d'un composé tel que défini dans la revendication 1 ou 2 pour une inhibition *in vitro* de DHODH.

10. Composition pharmaceutique à utiliser dans le traitement ou la prévention d'une maladie ou une indication choisie dans le groupe comprenant la fibrose, l'uvéite, la rhinite, l'asthme ou l'arthropathie, en particulier l'arthrose, toutes les formes de rhumatismes ; des événements et troubles immunologiques aigus tels que la sepsie, le choc septique, le choc endotoxique ; la sepsie Gram-négative, le syndrome du choc toxique, un syndrome de détresse respiratoire aigu, l'accident vasculaire cérébral, la lésion de reperfusion, la lésion du système nerveux central, des forme sérieuses d'allergie, des réactions greffe contre hôte et hôte contre greffe, la maladie d'Alzheimer ou la pyrésie, la resténose, une maladie inflammatoire pulmonaire chronique, la silicose, la sarcose pulmonaire, une maladie de résorption osseuse ; ces événements immunologiques comprennent également une modulation et une suppression souhaitées du système immunitaire ; tous les types de maladies auto-immunes, notamment la polyarthrite rhumatoïde, la spondylite rhumatoïde, l'arthrose, l'arthrite goutteuse, la sclérose en plaques, le diabète sucré insulinodépendant et le diabète non insulinodépendant, et le lupus érythémateux, la rectocolite hémorragique, la maladie de Morbus Crohn, une infection abdominale inflammatoire, ainsi que d'autres inflammations chroniques, la diarrhée chronique ; des troubles dermatologiques tels que le psoriasis, l'atrophie rétinienne progressive, tous les types d'infections, y compris les infections opportunistes, comprenant des composés tels que définis dans la revendication 1 ou 2.

11. Composition pharmaceutique à utiliser dans le traitement ou la prévention d'une maladie ou une indication choisie dans le groupe constitué par un rhumatisme, les troubles immunologiques aigus, les maladies auto-immunes, les maladies provoquées par une prolifération de cellules malignes, les maladies inflammatoires, les maladies provoquées par des infestations protozoaires chez l'être humain et l'animal, les maladies provoquées par des infections virales et *Pneumocycstis carinii,* la fibrose, l'uvéite, la rhinite, l'asthme et l'arthropathie, comprenant des composés tels que définis dans la revendication 1 ou 2.
